(19) Europäisches Patentamt
European Patent Office
Office européen des brevets

(11) EP 0 727 225 A2

(12) EUROPEAN PATENT APPLICATION

(43) Date of publication:
**21.08.1996 Bulletin 1996/34**

(51) Int Cl.[6]: **A61K 49/00**

(21) Application number: **96630007.1**

(22) Date of filing: **08.02.1996**

(84) Designated Contracting States:
**AT BE CH DE DK ES FR GB GR IE IT LI LU MC NL PT SE**

(30) Priority: **14.02.1995 US 388468**
**06.06.1995 US 471568**

(71) Applicant: **SONUS PHARMACEUTICALS, INC.**
**Bothell, WA 98021 (US)**

(72) Inventors:
- **Quay, Steven C.**
**Edmonds, Washington 98026 (US)**
- **Marrs, Christopher M.**
**Mukilteo, Washington 98275 (US)**
- **Worah, Dilip M.**
**Bothell, Washington 98011 (US)**

(74) Representative: **Schmitz, Jean-Marie et al**
**Dennemeyer & Associates Sàrl**
**P.O. Box 1502**
**1015 Luxembourg (LU)**

(54) **Compositions and methods for directed ultrasound imaging**

(57) Compositions for enhancing the ability to target gaseous microbubbles used in ultrasound contrast. The compositions include a cell adhesion molecule (CAM) ligand which is incorporated into a desired molecule to form a conjugate. Where the contrast agent is a collidal dispersion, the conjugate is formed with a surfactant. Where the agent is a solid microsphere, the conjugate is formed with the a portion of the solid. Once the conjugate is formed, the surfactant or microsphere will adhere to the surface of desired target cells by coupling of the CAM ligand to cell adhesion molecules expressed on the cell surface.

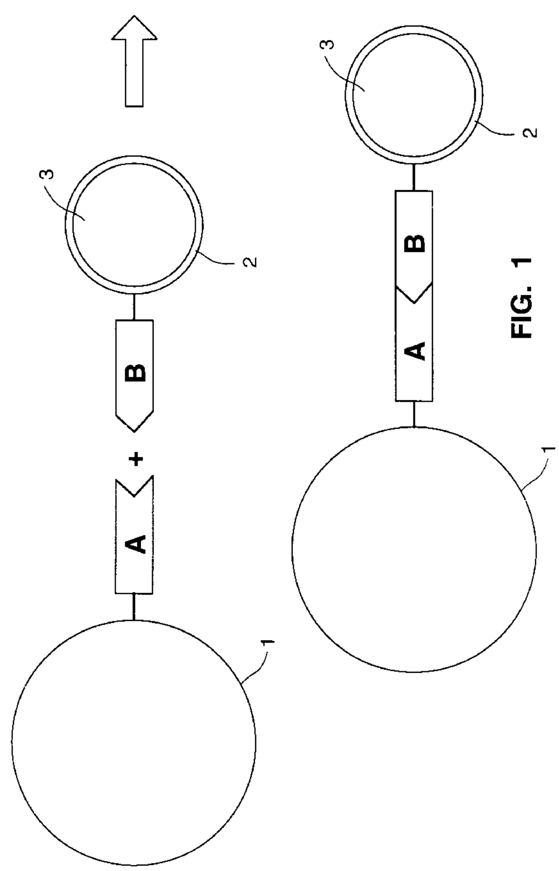

FIG. 1

EP 0 727 225 A2

**Description**

FIELD OF THE INVENTION

The present invention is directed to contrast agent compositions and the use of such compositions for in vivo ultrasound imaging. More specifically, the present invention is directed to a surfactant containing colloidal dispersions in which the surfactant has been modified to incorporate a cell adhesion molecule ligand. Using the dispersion so modified, the agents are directed to particular locations within the body, so that ultrasound contrast images from desired areas can be enhanced.

BACKGROUND OF THE INVENTION

Various contrast agents for use with diagnostic ultrasound, including echocardiography, have been described. Though research has intensified since, a review of the subject is found in Ophir and Parker, Ultrasound in Med. & Biol. (1989), 15:319-333.

In more recent years, agents which include gaseous microbubbles or microbubble precursors (as opposed to completely solid or liquid agents) have become the subject of great interest, since these agents take advantage of the relatively high echogenicity of gases versus liquids or solids. The difficulty with gaseous microbubbles has been at least two-fold: their relative instability and, as a related matter, the difficulty in timely moving the microbubbles to the area of interest.

To increase the persistence of microbubbles (to increase stability and therefore persistence in the body) numerous approaches have been disclosed. These include solid particulate carriers into which the bubbles are infused before use (e.g. U.S. 5,147,631), and encapsulants or-stabilizers for the microbubbles such as human serum albumin (e.g. U.S. 4,718,433).

U.S. patent application S.N. 07/936,011, having the same assignee as the present application, identifies methods of determining the relative persistence of biocompatible gases per se, thus identifying a group of compounds from which one can select highly persistent gases for use as microbubbles with many ultrasound agent carrier systems. Many of these compounds can be used as free gas microbubbles, that is, they are sufficiently persistent as gases that they do not require solid carriers or encapsulants to have commercial ultrasound imaging applications.

U.S. patent application S.N. 08/182,024, also assigned to the same assignee as the present application, discloses how a particular class of the compounds within those identified in S.N. 07/936,011 can be incorporated into a stable liquid-in-liquid dispersion. These compounds are liquids at common manufacturing temperatures, but gases in the body. This permits the manufacture of a liquid product which is highly stable prior to use, but which forms highly persistent microbubbles when used in the body.

The agents previously identified are proposed for many types of delivery to the body, most commonly intravenously. This is because of the intense commercial interest in imaging the heart in a fashion which is as non-invasive as possible. However, there are numerous other uses for such agents in other organs and systems of the body. For example, as is a well known goal of all contrast agents used for imaging in humans, it is highly desirable to be able to image potential or actual tumor sites. For many of these varied uses of imaging agents, it would be a significant advantage to identify a means other than simple direct application to the site in question, to identify a way to target a particular agent to a given site within the body. This is particularly true, keeping in mind that the specific location in question may not be known, for example a suspected tumor site. In any case, typical ultrasound contrast agents have, up to now, simply been applied directly to the area in question, such as the venous or lymph systems.

Identifying compositions which might be useful for targeting an ultrasound contrast agent thus involves many considerations, including identification of the mechanism by which targeting might be possible, biocompatibility of compositions which might vary from previous formulations, the ability to make such formulations in a storage stable form, cost and ease of use of the agent.

SUMMARY OF THE INVENTION

Thus, the present invention is directed to, in a first embodiment, an ultrasound contrast agent comprising microbubbles of biocompatible gas, a liquid carrier and a surfactant or solid (e.g., protein such as albumin) microsphere, wherein said surfactant or solid microsphere includes a cell adhesion molecule (CAM) ligand.

In another embodiment, the invention comprises an ultrasound contrast agent for use in an organism having a body temperature T comprising a liquid in liquid colloidal dispersion and a surfactant or solid microsphere, wherein a dispersed phase liquid comprises a chemical having a sufficiently high vapor pressure that a portion of said chemical is a gas at the body temperature T and wherein said surfactant or solid microsphere includes a CAM ligand.

In either of these embodiments, the surfactant may be a fluorine-containing surfactant and specifically a fluorine-containing and/or carbohydrate-based surfactant wherein the surfactant contains a CAM-ligand. In either of these embodiments, the CAM-ligand may bind to the GPIIb/IIIa receptor.

In an alternative embodiment, the CAM ligand is an KGD or RGD containing molecule.

In a further embodiment, the KGD or RGD containing molecule is selected from the group consisting of: trigramin, kistrin, albolabrin, elegantin, jarahagin, jaracetin, contortrostatin, multisquamatin, echistatin, eristocophin, eristostatin, albolabrin, applagin, barbourin, basilicin, batroxostatin, cerastin, ceraberin, cotiarin, crotatroxin, durissin, elegantin, flavoridin, halysin, jararacin, kistrin, lachesin, lutomin, moloosin, torgeminin, trigramin, viridin, bitistain, decorisn, mambin, ornatio E and halystatin.

In yet a further embodiment of the invention, the RGD containing peptide is a cyclic molecule having the structure:

$$X_1\ X_2\ X_3\ X_4\ G\ D\ X_5\ X_6\ X_7\ X_8$$

wherein $X_1$ and $X_8$ are 0 to 20 amino acids;
$X_2$ is 0 or an amino acid capable of forming a lactam bridge;
$X_3$ is 0 to 10 amino acids;
$X_4$ is a positive amino acid;
$X_5$ is a hydrophobic amino acid except leucine;
$X_6$ is a positively charged amino acid; and
$X_7$ is an amino acid capable of forming a lactam bridge.

In a further embodiment of the invention, the ultrasound contrast agent of further includes a second surfactant.

## BRIEF DESCRIPTION OF THE DRAWING

The invention will be better understood by reference to the appended drawing of which:

FIG. 1 is a diagram of the Avidin-latex biotin dodecafluropentane (DDFP) particle reaction scheme. A 1 micron latex microsphere **1** is attached to avidin (A) ; biotin (B) to the surfactant molecule **2** which contains DDFP **3**. Avidin (A) and biotin (B)interact to form the microsphere/avidin and biotin/DDFP complex.

## DETAILED DESCRIPTION OF THE INVENTION

This invention relates to specific in vivo targeting of ultrasound contrast agent compositions and methods for using such compositions in medical (both human and other animal) diagnosis. These agents are comprised of so-called CAM-ligands in combination with components used in known ultrasound contrast carriers. Specifically, such CAM-ligands can be conjugated with surfactants as an improvement to the agents disclosed in pending prior U.S. patent applications 07/936,011 and 08/182,024, each of which is incorporated by reference herein.

Thus, in its broadest form, the invention relates to the identification of molecules which can incorporate a CAM ligand, such that the conjugate formed will be attracted to cell adhesive molecules and thereby position the conjugate in higher concentrations at a desired cell surface.

In one application, the molecule which will form a conjugate with the ligand will be a surfactant, i.e. a molecule having a hydrophilic portion and a hydrophobic portion, so as to form a combination molecule comprising a hydrophobic portion, a hydrophilic portion and a CAM/ligand.

In a preferred use, such combination molecules can be used in a colloidal dispersion in which the dispersed phase is a liquid under the conditions of the manufacturing process (i.e. at standard temperature and pressure conditions) and which changes phase to become a dispersed gas or kugelschaum at or about the time of administration to the organism under study. The agents are "targeted" by conjugating a CAM ligand to the surfactant used in the dispersion. In this form, the CAM ligand, now associated with the surfactant at the surface of the microbubbles formed in use, will tend to bond to target cell adhesion molecules on the surface of desired cells, thereby targeting the ultrasound contrast agent to a specific receptor in vivo.

In this first embodiment, the liquid dispersed phase may be selected from the group of chemicals consisting of aliphatic hydrocarbons, organic halides or ethers, or combinations thereof, which have six or fewer carbon atoms and an upper limit of molecular weight of about 300. Among organic halides, the fluorine-containing chemicals are preferred, since they form stable emulsions and are relatively non-toxic. Especially preferred are n-pentane, isopentane, neopentane, cyclopentane, butane, cyclobutane, decafluorobutane, dodecafluoropentane, dodecafluoroneopentane, perfluorocyclopentane, perflurohexane and mixtures thereof. Preferably, the colloidal dispersion contains the dispersed phase at a concentration of 0.05 to 15.0% w/v. Optimally, the concentration range is 0.5 to 3.5% w/v.

The colloidal dispersion can be stabilized by the addition of various amphiphilic materials, including anionic, nonionic, cationic, and zwitterionic surfactants, which typically lower the interfacial tension between the dispersed liquid

and water to below 26 dynes/cm. Optimally, these materials are nonionic, synthetic surfactant mixtures, containing a fluorine-containing surfactant, such as the Zonyl brand series and a polyoxypropylene-polyoxyethylene glycol nonionic block copolymer. It is these amphiphilic materials which, in this embodiment, can be used to incorporate the CAM ligand.

The liquid continuous phase of the colloidal dispersion comprises an aqueous medium. This medium can contain various additives to assist in stabilizing the dispersed phase or in rendering the formulation biocompatible. Acceptable additives include acidifying agents, alkalizing agents, antimicrobial preservatives, antioxidants, buffering agents, chelating agents, suspending and/or viscosity-increasing agents, including triodobenzene derivatives, such as iohexol or iopamidol, and tonicity agents. Preferably, agents to control the pH, tonicity, and increase viscosity are included. Optimally, a tonicity of at least 250 mOsm is achieved with an agent which also increases viscosity, such as sorbitol or sucrose.

In another embodiment of the invention, the CAM ligand of the invention is associated with a surfactant, where the surfactant/CAM ligand combination is found at the interface between gaseous microbubbles and a liquid carrier phase, such that the surfactant, and therefore the microbubbles, will tend to accumulate at cell adhesion molecule sites for a given cell type.

In yet another embodiment of the invention, the CAM ligand is associated with a solid microsphere such as a proteinaceous microsphere, for example, the type sold as the Albunex brand contrast ultrasound contrast agent by Alliance Pharmaceuticals. In this embodiment, the ligand/albumin microsphere combination, like the surfactant/ligand combination of the first embodiment, will cause the microsphere to associate with cell adhesion molecules on the surfaces of desired cell types.

Thus, it will be understood that CAM ligands can usefully become part of the formulation of any ultrasound contrast agent in which a molecule which can bond with a CAM ligand is found and is associated closely enough with the agent itself to cause the agent to adhere to an adhesion molecule at the surface of a desired cell.

The terms used herein are to be defined in a manner consistent with the definitions and uses set forth in U.S. Pat. Application S.N. 07/936,011 and 08/182,024. To the extent any terms are used herein but not previously defined, or such definitions directly apply to the invention as claimed, such terms are defined as follows:

Surfactants: The group of amphiphilic materials which are manufactured by chemical processes or purified from natural sources or processes. These can be anionic, cationic, nonionic, and zwitterionic, and include the following distinct chemical groups:

Group 1: Acetamide Monoethanolamine (Mea), Acetylenic Diol, Acetylenic Diol Blend, Proprietary, Alcohol Alkoxylates, Alcohol And Alcohol Ether Sulfates, Alcohol-Ethoxylated-Propoxylated Surfactant Defoamer, Alcohol Ethoxysulfates, Alcohol, Oxyalkylated, Alcohol, Polyoxyethaylated Synthetic, Alcohols, Alkoxylated Linear, Alcohols, Detergent, Alcohols, Ethoxylated, Alcohols, Low-Foam Alkoxylated, Alcohol Sulfates, Aliphatic Alcohols, Ethoxylated Branched, Aliphatic Diamines, Aliphatic Ethoxylate, Linear, Aliphatic Nonionics, Alkanolamides, Alkoxylated Linear Alcohol Carboxylic, Acid Sodium Salts, Alkoxypolyalkoxyethanol, Alkyl Acid Phosphates, Alkyl Alkoxylate, Fluorinated, Alkylamine, Polyoxyethylated, Alkyl Amphoteric, Fluorinated, Alkylaryl Polyether, Alkylaryl Polyethoxylate-Sodium Salt Of Alkylsulfonatedalkylate Blend, Alkylaryl Polyoxyethylene Ether, Alkylaryl-Polyoxyethylene-Glycol, Phosphate Ester Surfactants, Solubilizers, Alkylaryl Polyoxyethylene Glycols, Alkylaryl Sulfonate;

Group 2: Alkylate Sulfonate, Linear, Alkylbenzenes, Alkyl Betaine, Alkyl Esters, Fluorinated, Alkyl Ether Sulfates, Alkyl Ethoxylate, Alkyl Imidazolines, Alkylolamides, Fatty Acid, Alkylolamides, Methyl Cocoate, Alkylphenol Alkoxylates, Alkylphenol Condensate, Ethoxylated, Alkylphenols, Ethoxylated, Alkylphenols, Polyoxyethylated, Alkyl Polyglycosides, Alkyl Quaternary, Fluorinated, Alkyl Sulfate, Alkyl Sulfate, Lauryl Alcohol, Amidoamine Methosulfate, Amidopropylamine Oxide, Amine Condensate, Amine Oxides, Amines, Primary, Ethoxylated, Amines, Tertiary, Ammonium Cumene Sulfonate, Ammonium Ether Sulfate, Ammonium Laureth Sulfate, Ammonium Lauryl Sulfate, Ammonium Lauryl Sulfosuccinate, Ammonium Xylene Sulfonate, Amphoterics;

Group 3: Amphoteric Salts, Anionic And Nonionic Surfactants, Anionic-Nonionic Blend Emulsifiers, Anionic-Nonionic Blends, Aromatic And Aliphatic Phosphate Esters, Avocadamine Dea And Avocado Oil, Betaines, Betaines, Amphoteric, Calcium Stearoyl 2-Lactylate, Capric Diethanolamide, Carboxylated Alkyl, Aryl-Alkyl Polyethoxylates, Castor Glycerides, Polyoxyethylated, Hydrogenated, Castor Oil, Ethoxylated, Castor Oil, Ethoxylated, Hydrogenated, Castor Oil, Refined Castor Oil, Sulfonated, Cationic Surfactant, Cetyl Acetate And Acetylated Lanolin Alcohol, Cocamide, Diethanolamine (Dea), Cocamide Monoethanolamine (Mea), Cocamidopropyl Amine Oxide, Cocamidopropyl Betaine, Cocamidopropyl Dimethylamine, Cocamphocarboxyglycinate, Cocoamine, Ethoxylated, Cocoamine Oxide, Cocoamine, Polyoxyethylated;

Group 4: Cocodiethanolamide, Coconut Acid Ester Of Sodium Isethionate, Coconut Amide Nonionics, Coconut Diethanolamide (68603-42-9), Coconut Monoethanolamide, Coconut Oil Diethanolamine Condensate, Cocoyl Imidazoline, Cocoyl Sarcosine, Cyclodextrins, Alpha, Beta, Gamma, Deceth-4 Phosphate, Decyl Alcohol, Ethoxylated, Decyl-Diphenyl Oxide Disulfonic Acid, Defoamer Blend, Demulsifiers (Emulsion Breakers), Diacetyltartaric Acid Esters Of Monoglycerides, Dialkyl(C12 C18) Dimethylammonium Chloride, Dicarboxylcocoimidazoline Compound, Diethanolamines, 2:1, Diethanolamine Lauryl Sulfate, Diethylene Glycol Monosterate, 3,5-Dimethyl-1-Hexyn-3-Ol, 3,6-Dimethyl-4-Octyne-3,6-Diol, Dimethyl Tertiary Amines, Dinonylphenols;

Group 5: Polyoxyethylated, Disodium Cocamido-Ethanolamine Sulfosuccinate (Mea), Disodium Cocamido Iso-Propanolamine Sulfosuccinate (Mipa), Disodium Ethoxylated Alcohol Half Ester of Sulfosuccinic Acid, Disodium Ethoxylated Nonylphenol Half Ester Of Sulfosuccinic Acid, Disodium Lauramido-Ethanolamine Sulfosuccinate (Mea), Disodium Laureth Sulfosuccinate, Disodium Oleamido-Ethanolamine Sulfosuccinate(Mea), Disodium Oleamido-Polyethyleneglycol-2-Sulfosuccinate, Disodium Oleamido-Iso-Propanolamine Sulfosuccinate (Mipa), Disodium Ricinoleamidoethanolamine Sulfosuccinate (Mea), Disodium Undecylenamido-ethanolamine Sulfosccinate (Mea), Dispersing Agents, Distearyldimethylammonium Chloride, Ditallowdimethylammonium Chloride, Dodecylbenzenesulfonic Acid;

Group 6: Dodecyldiphenylether Disulfonic Acid, Dodecyldiphenyl Oxide Disulfonic Acid, Dodecyl Diphenyl Oxide Sulfonate, Dodecylphenols, Emulsified Surfactant Defoamer, Emulsifiers, Emulsion Stabilizers, Esters, Ethanol-2-Phenolxy, Ether Sulfate, Ethoxylate, Complex, Ethoxylated Acetylenic Diol, Ethoxylated Alcohol Blends, Ethoxylated Alcohol Defoamer, Ethoxylated Derivatised Phenols, Ethoxylated-Emulsified Surfactant Defoamer, Ethoxylated Esters, Ethoxylated Fatty Acid Esters, Ethoxylated Fatty Alcohol Ethoxylated Lanolin Alcohols, Ethoxylated Polyoxypropylene Glycols, Ethoxylated-Propoxylated Block Copolymers Ethoxylated-Propoxylated Block Polymers, Ethoxylated-Propoxylated Sufactant Defoamer, Ethoxylated Sulfonate, Ethoxylated Surfactant Antifoam, Ethoxylated Surfactant Blend Defoamer, Ethoxylated Surfactant Defoamer;

Group 7: Ethoxylates, Nonionic, Ethoxylate Sulfate, Ammonium Salt, Ethoxylate Sulfate, Lauryl Alcohol, Ethylene Glycol Distearate, Ethylene Glycol Monostearate, Ethylene Oxide Adduct, Ethylene Oxide Condensate, Ethylene Oxide-Nonylphenol Adduct, Fatty Acid Alkoxylates, Fatty Acids, Polyoxyethylated, Fatty Alcohol-Ethylene Oxide Condensates, Fatty Alcohol Nonionic, Fatty Alcohol, Polyoxyethylated, Fatty Amides And Bisamides, Fatty Amine Alkoxylates, Fatty Diethanolamides, Fluorinated Surfactants, Fluoroalkyl Carboxylates, Fluorocarbons, Fluorosurfactants, Foamers, Glycerol Monooleate, Glycerol Monostearate, Hexadecyl Diphenylether Disulfonic Acid, Hydrotropes, Xylene, Imidazolines, Isoalcohol, Alkoxylated, Isopropylamine;

Group 8: Dodecyclbenzene Sulfonate, Isostearyl Alcohol, Alkoxylated, Isostearyl Lactate, Jojoba Oil Derivatives, Kerosene (Deodorized) Organic Defoamer, Lactamide Ethanolamine (Mea), Lanolin, Ethoxylated, Lauramide Diethanolamine (Dea), Lauramide Ethanolamine (Mea), Lauramide Nonionics, Lauramidopropyl Amine Oxide, Lauramidopropyl Betaine, Lauramidopropyl Dimethylamine, Lauramine Oxide, Lauric Acid, Ethoxylated, Lauric Acid Monoisopropanolamide, Lauric Diethanolamide, Lauric-Myristic Diethanolamide, Lauroyl Sarcosine, Lauryl Alcohol, Ethoxylated, Lauryl Monoethanolamide, Lauryl Polyglucose, Ligninamine, Lignin (Sulfonated), Sodium Salts;

Group 9: Linear Alcohols, Ethoxylated, Linoleic Diethanolamide, Methylbis- (Hydr.Tallowamidoethyl)-2-Hydroxyethylammonium Methyl Sulfate, Methylbis (Tallowamidoethyl)-2-Hydroxypropyl Ammonium Methyl Sulfate, Methyl-1-Oleylanidoethyl-2-Olelimidazolinium Methyl Sulfate, Methyl-1-Tallowamidoethyl-2-Tallowimidazolinium Methyl Sulfate, Mineral Seal Oil-Based Defoamer, Monocarboxylcocoimidazoline Compound, Monodiglycerides, Monoglyceride Citrate, Monoglycerides, Ethoxylated, Naphthalene-Formaldehyde Condensate (Sulfonated), Sodium Salt, Nonionic Surfactant Nonyldiphenyl Ether Disulfonic Acid, Nonylphenol Ethoxylate, Nonylphenol Nonionics, Nonylphenols, Polyoxyethylated;

Group 10: Nonylphenoxypoly(Ethyleneoxy) Ethanol (Sulfated), Ammonium Salt, Octylphenol Ethoxylate, Octylphenols, Polyoxyethylated, Octyl Salicylate, Oleamide Diethanolamini (Dea), Oleamidopropyl Dimethylamine, Oleic Acid,Ethoxylated Oleic Diethanolamide, N-Oleoylsarcosine, Oleyl Alcohol Phosphate Ester, Oleyl Alcohol, Polyoxyethylated, Oleylamine,Ethoxylated, Organic Phosphate Esters,Free Acids Of , Organic Salt, Organic-Silicone Blend, Antifoam, Organic-Silicone Defoamer, Oxazolines, Paper Additives, Peg-15 Cocamine Phosphate Oleate, Perfluoroalkyl Sulfonates, Phosphate Acid Esters, Aliphatic Base, Phosphate Acid Ester, Aromatic Base, Phosphate Acid Esters, Aromatic Hydrophobic Base, Phosphate Acid Esters, Fatty Alcohol, Phosphate Acid Esters, Linear Alcohol, Phosphated Alcohol Ethoxylate, Phosphate Ester, Aliphatic Hydrophobic Base;

Group 11: Phosphate Ester-Free Acids, Phosphate Ester, Partial Sodium Salt, Phosphate Esters, Phthalic Glycerol Alkyd Resin, Modified, Polyacrylic Acid, Polyalkylene Oxide, Polyether, Alkoxylated, Polyether, Block Polymer, Polyethers, Folyethoxylated Amines, Polyethoxylated Fatty Acids, Polyethylene Emulsions, Polyethylene Glycol Dioleates, Polyethylene Glycol Ditallate, Polyethylene Glycol Esters, Polyethylene Glycol Monolaurate, Polyethylene Glycol Monooleate, Polyglycerol Esters, Polyoxyethylene Caster Oil, Polyoxyethylene Cocoamine, Polyoxyethylene Oleic Acid, Polyoxyethylene Stearic Acid, Polyoxyethylene Tallowamine, Polypropylene Glycol Distallate;

Group 12: Polypropylene Glycol Ester, Polysodium Vinylsulfonate, Potassium And Sodium Soaps, Potassium Cocoates, Potassium Toluenesulfonate, Propoxylated Alcohol, Propoxylated Polyoxyethylene Glycols, Pyridine-3-Sulfonic Acid, Quaterinaries, Quaternary Alkylamines, Quaternary Ammonoim Compounds, Quaternary Ammonium Salts, Quaternary Biocides, Ricinoleamide Diethanolamine (Dea), Ricinoleic Diethanolamide, Silicone Antifoams, Silicone Coatings, Silicone Defoamers, Silicone Emulsions, Silicone Fluids, Silicone-Glycol Copolymers,

Sodium Alkylarylsulfonate Sodium Alkylnaphthalenesulfonate, Sodium Bistridecyl Sulfosuccinate, Sodium Butoxyethoxy Acetate, Sodium Capryl Lactylate, Sodium N-Cocoyl-N-Methyltaurate;
Group 13: Sodium Cocoylsarcosinate, Sodium Cumenesulfonate, Sodium Decyldiphenyl Ether Sulfonate, Sodium Diamyl Sulfosuccinate, Sodium Dibutyl Sulfosuccinate, Sodium Dicarboxyethylcoco Phosphoethyl Imidazoline, Sodium Dicyclohexyl Sulfosuccinate, Sodium Dihexyl Sulfosuccinate, Sodium Diisobutyl Sulfosuccinate, Sodium Dioctylsulfosuccinate, Sodium Dioctylsulfosuccinate And Mineral Spirits, Sodium Dioctylsulfosuccinate And Propylene Glycol, Sodium Dodecylbenzenesulfonates, Sodium Dodecyldiphenyl Ether Sulfonate, Sodium 2-Ethylhexyl Sulfate, Sodium Isodecyl Sulfosuccinate, Sodium Isostearoyl Lactylate, Sodium Laureth Sulfate, Sodium Lauroyl Lactylate, Sodium Lauroylsarcosinate, Sodium Lauryl Sulfate, Sodium Lauryl Sulfoacetate, Sodium N-Methyl-N-Oleoyltaurate, Sodium Naphthalene Sulfonate, Sodium 1-Octane Sulfonate, Sodium C14-16 Olefin Sulfonates, Sodium Polyacrylate, Sodium Stearoyl Lactylate, Sodium Tetradecyl Sulfate, Sodium Toluenesulfonate, Sodium Toluene-Xylenesulfonate, Sodium Vinylsulfonate, Sodium Xylene Sulfonate, Sorbitan And Ethoxylated Sorbitan Esters, Sorbitan Oleate, Sorbitan Stearate, Soya Amine;
Group 14: Polyoxyethylated, Soyamide Diethanolamine (Dea), Stearalkonium Chloride, IsoStearamidopropyl Bentaine, Stearamidopropyl Dimethylamine, Stearamidopropyl Pg-Dimonium Chloride Phosphate, Stearic Acid Diethanolamide, Stearic Acid, Ethoxylated; and
Group 15: Stearic Acid Monoethanolamide, Stearic Imidazoline, Stearylamine,Ethoxylated, Succinimides,Basic, Sucrose Esters, Sulfate Ester, Sulfates And Ether Sulfates, Sulfobetaines, Sulfonates, Sulfonates,Dodecylbenzene, Sulfonic Acid, Linear Alkyl(C-12) Benzene, Sulfosuccinamates, Sulfosuccinate Esters, Sulfosuccinates, Surfactant, Oil Defoamer, Surfactant Solution Defoamer, Surfactants, Low-Foaming, Surfactants, Soluble-Oil-Base, Polyoxyethylated Tall Oil Fatty Acid, Ethoxylated Tall Oil Fatty Acids, Tall Oil Imidazoline, Ethoxylated Tallowamine, Polyoxyethylated Tallowamine, Dihydrogenated Tallowdimethylammonium Chloride,, Dihydrogenated Tallowdimethylammonium Methyl Sulfate, Tetrahydroxypropylethylenediamene, Tert-Thioethoxylate, Toluenesulfonic Acid, Ethoxylated Tridecyl Alcohol, Tridecyloxypoly-(Ethyleneoxy)-Ethanol, Triethanolamine Lauroylsarcosinate, Triethanolamine Lauryl Sulfate, Triethanolamine Phosphate Ester, Trimethylnonyl Ether Of Polyethylene Glycol, Wetting Agents, Yucca Extract.

Amphiphilic Material: A substance which is strongly adsorbed at an interface and which normally produces a dramatic reduction in the interfacial tension with small changes in the bulk phase concentration. Examples include synthetic surfactants, naturally occurring materials such as biocompatible proteins, lipids, sterols, alginates, cellulose derivatives, and finely divided organic or inorganic particulate solids.

Polyoxypropylene-Polyoxyethylene Glycol Nonionic Block Copolymers: The surfactants which are available from BASF Performance Chemicals, Parsippany, New Jersey under the trade name Pluronic and which consists of the group of surfactants designated by the CTFA name of poloxamer 108, 188, 217, 237, 238, 288, 338, 407, 101, 105, 122, 123, 124, 181, 182, 183, 184, 212, 231, 282, 331, 401, 402, 185, 215, 234, 235, 284, 333, 334, 335, and 403.

Fluorine-Containing Surfactant: A surfactant containing one or more fluorine molecules. Some but not necessarily all fluorine containing surfactants, useful in this invention can be selected from the group consisting of: telomer B containing fluorinated surfactants available from Du Pont, Wilmington, DE under the Trade name of Zonyl (including Zonyl FSA, FSP, FSE, UR, FSJ, FSN, FSO, FSC, FSK, and TBS), the fluorochemical surfactants from 3M Industrial Chemical Products Division, St. Paul, MN under the trade name of Fluorad (including FC-95, FC-98, FC-143, FC-170C, FC-171, FC-430, FC-99, FC-100, FC-120, FC-129, FC-135, FC-431, FC-740), the perfluoroalkylpoly(oxyethylene) surfactants described by Mathis et al. (J Am Chem Soc 106, 6162-6171 (1984), incorporated herein by reference), the fluoroalkylthio-etherpoly(oxyethylene) surfactants described by Serratrice et al. (J Chim Phys 87, 1969-1980 (1990), incorporated herein by reference), the perfluoroalkylated polyhydroxylated surfactants of Zarif et al. (J Am Oil Chem Soc 66, 1515-1523 (1989), incorporated herein by reference), the fluorosurfactants available from Atochem North America, Philadelphia, PA under the trade name of Forafac.

Fluorine-Containing Carbohydrate Based Surfactant : A carbohydrate-based surfactant containing one or more fluorine molecules. Fluorine containing surfactants are a particularly useful class of surfactants for stabilizing colloidal dispersions of the type which may be used in the present invention. Where such surfactants include carbohydrate and/or peptides, the CAM ligand of the invention can be incorporated in or conjugated with the surfactant. Such surfactants are disclosed in: Le Blanc et al., "Deviation from Molecular Aging Model in Fluorocarbon Emulsions Stabilized by Perfluoroalkylated Surfactants ," J. Colloid and Interface Science, Vol. 137, No. 2, 1990; Greiner et al., "Synthesis and Preliminary Evaluation of 2-(F-Alkyl)-Ethyl Glycosides," New Journal of Chemistry, Vol. 13, No. 3, 1989; and Milius et al., "Synthesis of F-Alkylated Glycosides as Surfactants for In Vivo Uses..." New Journal of Chemistry, Vol. 15, No. 5, 1991, each of which is incorporated herein by reference.

Microspheres: Microspheres are solid or semisolid carriers including nanospheres, microballons, liposomes and the like which may contain chemicals for pharmaceutical use including drug delivery, medical imaging, oxygen delivery and the like. Microspheres may be comprised of protein, biodegradable polymers such as those disclosed in WO/9317718, Schneider, et al., Invest Radiol. 27:134-139 (1992), Gref, et al. Science 263: 1600-03 (1994), EP 324-938 (1988) and EP 90810367 (1990) which are hereby incorporated by reference and other amphilic materials.

Antagonists: Antagonists are molecules which block binding of agonists to a receptor or receptor (s).

Cell Adhesion Molecules: Cell adhesion molecules (CAMs) are molecules selected from the group consisting of cytokines, selectins, integrins, immunoglobulin (Ig) superfamily and cadhedrins.

Cytokines: Cytokines are cellular regulatory proteins. They are produced by specific cells in response to a variety of stimuli and they influence the behavior of target cells. They may act systemically or locally. Examples of cytokines include: Angiogenin, Epidermal growth factor, erythropoietin, Fibroblast Growth Factor (FGF), FGF basic, FGF basic, FGF-3, FGF-4, FGF-5, FGF-6, FGF-7, granulocyte-colony stimulating factor, granulocytemacrophage colony stimulating factor, GROα/MGSA, hepatocyte growth factor, heparin binding epidermal growth factor, interferon (IFN), IFNα/B, IFNδ, insulin-like growth factor (IGF), IGF-I, IGF-II, interleukin (IL), IL-1α, IL-1β, IL-1ra, IL-2, IL-3, IL-4, IL-5, IL-6, IL-7, IL-8, IL-9, IL-10, IL-11, IL-12, IL-13 and IL-14.

Selectins. immunoglobulin (Ig) superfamily and cadhedrins: Selectins, immunoglobulin (Ig) superfamily and cadhedrins are expressed on cell surfaces and are involved in cell adhesion. Selectins have been implicated in the initial interactions between leukocytes and vascular endothelia leading to lymphocyte homing, platelet binding and neutrophil extravasation. Examples of selectins include E-selectins, L-selectins and P-selectins. Examples of immuglobulin superfamily: Intercellular Adhesion molecule (ICAM), ICAM-1, ICAM-2, ICAM-3, Vascular cell adhesion molecule (VCAM), VCAM-1, VCAM-1(alt), platelet endothelial cell adhesion molecule and mucosal addressin cell adhesion molecule. Examples of cadhedrins include CD44/H-CAM/GP90-Hermes.

Integrins: Integrins are heterodimeric glycoprotein receptors that share a common β-subunit which combines noncovalently with a unit α-subunit to create functionally distinct receptors. Their role is to integrate the ECM outside the cell with actin containing cytoskeleton inside the cell.

Integrin receptors are involved in adhesion in thrombus formation and restenosis, growth, tumor cell recognition and metathesis of cancer cells, angiogenesis, autoimmune diseases, infection and inflammation. Thrombus formation is dependent on platelet aggregation. Platelet aggregation is in turn dependent on the binding of fibrinogen and other serum proteins to the integrin glycoprotein receptor IIb/IIIa located on the platelet plasma membranes.

Other examples of integrins include the VLA family: VLA-11, VLA-2, VLA-3, VLA-4, VLA-5, VLA-6, βIα7, βIα8, BIαv; the LEUCAM family: LFA-1, Mac-1 and p150.95; and the cytoadhesin family: CD41a, vitronectin receptor, $\beta_4\alpha_6$, $\beta_5\alpha_v$; $\beta_6\alpha_V$, $\beta_7\alpha_4$, LPAM-1, $\beta_7\alpha_{IEL}$, $\beta_8\alpha_\delta$ and others.

Cell Adhesion Molecule Ligands: Cell adhesion molecule ligands (CAM ligands or CAMLIG) or antagonists are molecules which specifically interact with cell adhesion molecules. CAM ligands useful in the present invention include oligosaccharides terminating in the Sialyl Lewis X (SLex) sequence: α-Sialic-acid (2→3) βGa1 (1→4)[αFuc(1→3)βGlc-NAc-OR which bind to a number of selectins; MAdCAM-1, CD34 and GlyCAM-1 which bind to L-selectins; PSGL-1 which bind to a number of P-selectins; LFA-1, and Mac-1 which bind to various ICAM's; VLA4 which binds to VCAM-1; PECAM-1 which binds to PECAM-1.

Other CAM ligands useful in the invention include Arg-Gly-Asp (RGD) containing peptides. Many integrin receptors recognize and bind their glycoprotein ligand at sites containing the amino acid sequence Arg-Gly-Asp (RGD). The importance of the RGD motif for cell attachments was first established in fibronectin and has since been identified in the cell recognition site of vitronectin, fibrinogen, von Willebrand factor, type I collagen, thrombospondin and osteopontin. Recently hepatocyte attachment to laminin, human neutrophil (PMN) phagocytosis and the adhesion of glilmesenchymal extracellular matrix glycoprotein Tensacin have been demonstrated to be accomplished by RGD. High levels of RGD binding are associated with cardiovacular disease, cancer, osteoporosis and inflammation.

The RGD sequence takes on different conformations in different proteins. The affinity of such peptides depends significantly upon their secondary and tertiary structure. Cyclic RGD peptides and their mimetics have achieved nanomolar affinity for the GPIIb/IIIa receptor. Such cyclic molecules includes those having the structure:

$$X_1\ X_2\ X_3\ X_4\ G\ D\ X_5\ X_6\ X_7\ X_8$$

wherein $X_1$ and $X_8$ are 0 to 20 amino acids;
$X_2$ is 0 or an amino acid capable of forming a lactam bridge;
$X_3$ is 0 to 10 amino acids;
$X_4$ is a positive amino acid;
$X_5$ is a hydrophobic amino acid except leucine;
$X_6$ is a positively charged amino acid; and
$X_7$ is an amino acid capable of forming a lactam bridge.

Specific peptides include:

GNPRGD (O-n-butyl-Y)RE-$NH_2$
GRGD (O-Me-Y)RE-$NH_2$
GNPRGD (O-Me-Y)RE-$NH_2$
GNPRGD (O-Me-Y)RD-$NH_2$
($\beta$-Ala)RGD(O-Me-Y)RD-$NH_2$
($\beta$-Ala)NPRGD(O-Me-Y)RD-$NH_2$
GRGD(O-n-butyl-Y)RE-$NH_2$.
Ac-CRGD(O-Me-Y)RC-OH
Ac-CRGD(O-Me-Y)R(Pen)-NH2
Ac-(dPen)RGD(O-Me-Y)RC-$NH_2$
Ac-CKGD(O-Me-Y)RC-$NH_2$
Ac-CRGD(O-Me-Y)R(dPen)-$NH_2$
Ac-CRGD(O-Me-Y)RC-$NH_2$
Ac-CRGD(O-Me-Y)RC-R-$NH_2$
(Mpr)RGD(O-Me-Y)Rc-$NH_2$
(Mpr)RGD(O-n-butyl-Y)RC-$NH_2$
(Mpr)KGD(O-Me-Y)Rc-$NH_2$
(Pmp)RGD(O-Me-Y)Rc-$NH_2$
Ac-CRGD(O-n-butyl-Y)RC-$NH_2$ and
Ac-CRGD(Hpa)RC-$NH_2$

as disclosed in WO 95/00544 which is hereby incorporated by reference.

Some peptides foreign to the immune system also take advantage of this binding ability. They include the snake venoms, annexin and potato lectin. Snake venoms, deriving from the *Viperidae* family, have been shown to have extremely high affinity for the GPIIb/IIIa receptor, (greater than that of fibronectin). These molecules contain an RGD or KGD sequence. These molecules include, among others, Echistatin, Trigramin, Bitistatin, Kistrin, Albolabrin, Flavoviridin, Barbourin, Elegantin, Cotiarin, Jarahagin, Jaracetin, Contortrostatin, Multisquamatin and Halystatin.

In addition to RGD, other peptide fragments are implicated in cell adhesive phenomena: KGD, found in Barbourin; SPENP, found in Elegantin and von Willebrand factor; CS-1, its core, cyclic, and optimized peptide sequences which are responsible for selective VLA-4 inhibition; PRARI, implicated in heparin binding domain and focal adhesion formation and Disagregin which is involved in fibronectin binding.

CAM ligands consisting of cyclic compounds containing carbocyclic or heterocyclic ring systems which act as antagonists of the GPIIb/IIIa receptor are described in WO 94/22494 which is hereby incorporated by reference.

Antibodies including monoclonal and polyclonal antibodies to the GPIIb/IIIa receptor and peptides containing these sequences or a peptidomimetic equivalent of these sequences are also antagonists of the integrin receptors. Non-RGD integrin ligands include collagen, specific for $\alpha_1\beta_1$, $\alpha_2\beta_1$ and $\alpha_3\beta_1$, laminin and kalinin, specific for $\alpha_2\beta_1$, $\alpha_3\beta_1$ and $\alpha_6\beta_1$. V-CAM, specific for $\alpha_4\beta_1$; ICAM-1 and I-CAM-2, specific for $\alpha_L\beta_1$ and $\alpha_M\beta_1$. There are also non-RGD binding regions of fibronectin and fibrinogen.

Other peptides which may serve as CAM ligands in the invention include those disclosed in WO 94/07918, WO 94/19024, WO 93/25244, WO 93/23085 and WO 94/28942 which are hereby incorporated by reference.

Attachment of these molecules to ultrasound contrast agents either directly or with a covalent or non-covalent tether will provide for site-directed ultrasound contrast whose selectivity is related to the binding coefficient and specificity of the ligand.

In the invention, a surfactant of the type known for stabilizing a colloidal dispersion, where a portion of the dispersed phase is or will become gaseous at the body temperature of an animal is modified to incorporate a CAM ligand. This modified surfactant can be called a conjugated CAM ligand/surfactant molecule.

To achieve this end, in a preferred embodiment, the surfactants are first purified by dissolving them in organic solvents such as ethyl acetate, acetone, acetonitrile, toluene, tetrahydrofuran, methylene chloride, dichloroethane, water, or most preferably with a normal or branched alcohol ($C_1$-$C_5$) of maximum solvent polarity or any mixture of the above solvents, adding activated charcoal (1-1000% by weight) and heating the solvent (25-140°C) and filtering off the charcoal. The charcoal can be in powder, granular form, embedded onto a support such as cellulose or encased in a cartridge in order to effect purification. Removing the solvents *in vacuo* provides for a composition in which the color and the volatile components are reduced.

An alternative procedure for reducing the color and volatile impurities is to distill the surfactant composition (80-300°C) under high vacuum (10-200 microns Hg).

The following two methods reduce the volatile components further. These methods reduce the volatile components to part per million levels but reintroduce substantial amounts of color to the surfactant composition.

Conducting the surfactant through a heated glass apparatus, such as a falling film apparatus or other device, which

has been modified to allow the passage of gas, reduces the volatile components of fluorinated surfactants further. The apparatus is heated (50-200°C) and an inert gas such as helium, neon, perfluorocarbon ($C_1$-$C_5$), Freon 11-245ea, (b. p. <50°C), argon, nitrogen, sulfur hexafluoride or air is passed through (10-150 L/min). The material is conducted through such apparatus until the volatile components have been reduced to the desired level.

The volatile components of fluorinated surfactants may also be reduced by placing the material under high vacuum (1-1000 microns Hg) and heat (0-200°C) until the volatile components reach the desired levels.

The purified surfactants are reacted with an "activating group" or conjugating fragment. The combined surfactant and fragment will then react with the CAM ligand to form the final conjugated CAM ligand/surfactant molecule.

Oligosaccharides referred to as "Lewis" molecules are examples of a molecule type which selectively binds to E-Selectin, that is, act as a CAM ligands. A series of such compounds such as Lewis X, Lewis a, 3'- Sialyl Lewis X, 3'-Sialyl Lewis a, 3" Sulfated Lewis X are available from Oxford Glycosystems of Rosedale, N.Y. These compounds can be conjugated to fluorinated surfactants by using activated fragments, where "activated" refers to the use of a functional group on the surfactant fragment which will react with a corresponding group on the CAM ligand to form a covalent bond, and the term "fragment" is used to mean the portion of the surfactant molecule involved in this reaction.

Those skilled in the art will understand that the specific choice of a fragment will necessarily depend on the particular CAM ligand that is to be used, or at least the active group on the ligand to which the fragment may bond.

The invention will be better understood by way of the following examples, however, it is to be understood that these examples are illustrative only and are not to be construed as limiting the invention as claimed below:

## Example 1

This example shows the purification of surfactants for use in the preparation of CAM ligand conjugates.

### Preparation Method 1

150g Zonyl FSO-100 is distilled from 90-240°C under high vacuum (30-80 microns Hg) to yield 110g (73%) pale yellow oil.

### Preparation Method 2

40g Zonyl FSN-100 and 80g activated charcoal are dissolved in 600mL isopropyl alcohol heated to boiling and filtered. The solvent is concentrated *in vacuo* to yield 18.1g (45% as a clear oil which solidifies upon standing.

### Preparation Method 3

200g Flourad F-170C and 400g activated charcoal are suspended in 1500mL of ethyl acetate. The solution is brought to boiling and filtered. The surfactant is concentrated *in vacuo* to yield 104.2g (52%) of a pale yellow oil.

### Preparation Method 4

300g Zonyl FSN-100 and 375g activated charcoal are dissolved in 1500mL methyl alcohol, heated to boiling and filtered. The charcoal is resuspended in 1200mL of methanol, heated to boiling and filtered. The solvent is concentrated *in vacuo* to yield 203g (68%) of a light yellow, milky oil.

## Devolatilization

### Preparation Method 5

16g Decolorized Zonyl FSN-100 is dripped into an inclined heated tube heated with 100°C glycol/water, through which nitrogen is conducted at 25L/min, to yield 13.5g (85%) colorless oil.

### Preparation Method 6

60g Decolorized Fluorad F-170C is placed in a 100mL round bottom flask with stir bar. The flask is slowly evacuated to full vacuum and the heat is brought to 50°C. The surfactant is treated in this way for 36 hours when the vacuum had reached 30 micron. 40.2g (67%) dark red oil was obtained.

Preparation Method 7

100g Afilan OTN is run through a falling film apparatus heated by nonane heated at reflux. Nitrogen gas, preheated to 50°C, is passed through the apparatus at 85 L/Min. 77.8g (78%) yellow oil was obtained.

Preparation Method 8

The decolorized Zonyl FSO-100, from purification method 4, was in placed in a 250mL round bottom flask with stir bar. The flask is slowly evacuated to full vacuum and the heated at 50°C for 24 hours until the vacuum had reached 19 micron. 196g (65%) of a light yellow milky oil was obtained.

Preparation Method 9

300 g Zonyl FSO-100 and 375 g activated charcoal are suspended in 1500 ml methanol, heated to boiling and filtered *in vacuo.* The filtrates are combined and concentrated *in vacuo.* The decolorized material is placed under high vacum (30 microns Hg) and stirred at 50°C for 72 hours. A pale yellow oil, 195 g with white precipitate is obtained (65% yield).

| Purification Evaluation Methods | | | | |
|---|---|---|---|---|
| Visible Spectrum 400-700nm 10% In Methanol | Extinction coefficient(a) @ 400nm | | | |
| | Crude | | Decolorized | |
| Preparation Method 1 Zonyl FSO-100 | 9.865 | | 1.956 | |
| Preparation Method 2 Zonyl FSN-100 | 11.327 | | 3.800 | |
| Preparation Method 3 Fluorad F-170C | 4.519 | | 4.989 | |
| Preparation Method 4 Zonyl FSO-100 | 9.865 | | 1.517 | |
| Crude Afilan OTN | 1.755 | | 1.517 | |
| | | | | |
| Gas Chromatography | Dioxane (ppm) | | Total Volatiles (ppm) | |
| | Crude | Devolatilized | Crude | Devolatilized |
| Preparation Method 1 Zonyl FSO-100 | 1213 | 27 | 3315 | 637 |
| Preparation method 4 Zonyl FSN-100 | 914 | <1 | 3677 | 347 |
| Preparation Method 5 Fluorad F-170C | 1478 | <1 | 3559 | 243 |
| Preparation Method 6 Afilan OTN | 25 | <1 | 2677 | 26 |
| Preparation Method 7 Zonyl FSO-100 | 1213 | 4.92 | 3315 | 672 |

Example 2

This example shows the preparation of CAM ligand/surfactant conjugates which can then be incorporated into the contrast agent. These reactants show the varied types of functional groups which can be used to "activate" the surfactant. For each reaction shown, $R_f = C_nF_{2n+1}$ and CAMLIG=cell adhesion molecule ligand.

1. $R_f(CH_2CH_2O)_xCOCl$ + CAMLIG-$NH_2$ $\rightarrow$ $F_f(CH_2CH_2O)_xCONH$-CAMLIG

2. $R_f(OCH_2CH_2)_xCOCl$ + CAMLIG-OH $\rightarrow$ $R_f(CH_2CH_2O)_xCO_2$-CAMLIG

3. $R_fCH_2CH_2(OCH_2CH_2)_xSH$ + CAMLIG-SH+1/2O2 $\rightarrow$ $R_fCH_2CH_2(OCH_2CH_2)_xSS$-CAMLIG

4. $R_fSO_2Cl$ + CAMLIG-$NH_2$ $\rightarrow$ $R_fSO_2NH$-CAMLIG

5. CAMLIG-CHO + $R_fCH_2CH_2(OCH_2CH_2)_xNH_2$ + $NaCNBH_3$ $\rightarrow$ $R_fCH_2CH_2(OCH_2CH_2)_xNH$-CAMLIG

6. $CAMLIG\text{-}Br + R_fCH_2CH_2(OCH_2CH_2)_xSH \rightarrow R_fCH_2CH_2(OCH_2CH_2)_xS\text{-}CAMLIG$

7. $CAMLIG\text{-}Br + R_fCH_2 + Bu_3SnH \rightarrow R_fCH_2CH_2\text{-}CAMLIG$

8. $R_fCOCl + CAMLIG\text{-}NH_2 \rightarrow R_fCONH\text{-}CAMLIG$

9. $R_fNCO + CAMLIG\text{-}NH_2 \rightarrow R_fNCONH\text{-}CAMLIG$

10. $CAMLIG\text{-}CHO + R_fCH_2CH_2(OCH_2CH_2)_xNH_2 \rightarrow R_fCH_2CH_2(OCH_2CH_2)_xNH\text{-}CAMLIG + R_fCOCl \rightarrow$

$$(R_fCH_2CH_2(OCH_2CH_2)_x)\ (R_fCO)N\text{-}CAMLIG$$

It is expected that Sialyl Lewis X can be conjugated with many other types of surfactants, for example, poloxamers, polyethylene glycols, fatty acids, fatty amines, fatty alcohols and phosphatidyl choline.

With respect to polyethylene glycol containing fragments, the following can be used: PEG2 NHS ester, NHS-PEG-VS, NHS-PEG-MAL, Methoxy-PEG-vinylsulfone, PEG- $(VS)_2$, Methoxy-PEG-ald, PEG-$(ald)_2$, Methoxy-PEG-epx, PEG-$(epx)_2$, Methoxy-PEG-Tres, PEG-$(Tres)_2$, Methoxy-PEG-NPC, PEG-$(NPC)_2$, Methoxy-PEG-CDI, PEG-$(CDI)_2$, mPEG-Gly-OSu, mPEG-NLe-OSu, methoxy-SPA-PEG, $(SPA)_2$-PEG, Methoxy-SS-PEG, $(SS)_2$-PEG all of which are available from Shearwater Polymers, Inc. of Huntsville, Alabama, U.S. Where these types of fragments are used, i.e. where the fragments may not themselves have surfactant properties adequate for a given ultrasound contrast formulation, or act only weakly as surfactants, the conjugate formed can be used in conjunction with other surfactants in the final formulation.

Once the CAM ligand is effectively joined to the fragment, the conjugate/surfactant combination can be incorporated in the ultrasound contrast agent formulation, as is known.

Example 3

This example shows the preparation of a fluorine containing carbohydrate based surfactant of the type which can be used to make conjugated surfactant/CAM ligands useful in colloidal dispersions, and which, therefore, may have application in the formulation of ultrasound contrast agents which utilize such surfactants.

$$C_9F_{19}CH_2CH_2(OCH_2CH_2)_{\text{-}9}\text{-}OH + Cl_3CCOCOCCl_3 \rightarrow$$

$$C_9F_{19}CH_2CH_2(OCH_2CH_2)_{\text{-}9}OCOCl + \text{glucosamine HCL} + \text{Pyridine}$$

$$\rightarrow C_9F_{19}CH_2CH_2(OCH_2CH_2)_xOCONH\text{-glucosamine}$$

Peg Telomer B, (0.887g. - 1 mmol) is dissolved in 2 mL $CH_2CH_2$ in a 10 mL round bottom flask. To which triphosgene, (0,324g 1.1 mmol) is added. The reaction is let stir for 24h. To which glucosamine hydrochloride, (.634g 1.1 mmol) is added followed by pyridine, (142µL, 1.6 mmol). The reaction is let sit for an additional 48h. TLC with 98:2 ethyl acetate/ water showed complete conversion to product. Removal of the solvent in vacuo provided a red oil.

Example 4

This example shows the preparation of a another fluorine containing carbohydrate based surfactant of the type which can be used to make conjugated surfactant/CAM ligands useful in colloidal dispersions, and which, therefore, may have application in the formulation of ultrasound contrast agents which utilize such surfactants.

$$\text{N-methyl glucamine} + C_8F_{17}SO_2Cl \rightarrow \text{N-perfluorooctasulfonyl glucosamine}$$

N-methyl glucamine, (10g, 51.2 mmol), is dissolved in 100 mL dimethylacetamide. To which perfluorooctasulfonylchloride, (6g, 11.6 mmol) is added. The reaction is let sit 16h. The white precipitate is filtered off, washed with methylene chloride. The solvent is concentrated in vacuo and chromatographed with 86% methylene chloride, 10% acetonitrile and 4% methanol with ammonia. 2.3g pure white crystalline solid obtained after concentration of the fractions, 30% yield.

Example 5

These examples (5-8) show the preparation of conjugates of Sialyl Lewis X with various surfactants as identified:

$NeuNAc\alpha 1\text{-}3Gal\beta 1\text{-}4GlcNAc + MeO(CH_2CH_2O)_{45}CH_2CHNH_2 + NaCNBH_3 \rightarrow$

3 $CH_3$

Sialyl Lewis X Fucα1 Jeffamine M-2070

$NeuNAc\alpha 1\text{-}3Gal\beta 1\text{-}4GlcNAc1\text{-}NHCHCH_2(OCH_2CH_2)_{45}OMe$

3 $CH_3$

Fucα1

Jeffamine M-2070, (504mg, 0.243mmol), Sodium Cyanoborohydride (8.6mg, 0.1365mmol) and Acetic acid (14.6mg 0.243mmol) are dissolved in 1mL of water. 1μL of this solution is added to 3' Sialyl Lewis X. Reaction is allowed to stand for 24h at room temperature.

Example 6

$MeO(CH_2CH_2O)_{45}CH_2CHNH_2 + CCl_3OCOCCl_3 \rightarrow MeO(CH_2CH_2O)_{45}CH_2CHNHCOCl$

$CH_3$ $CH_3$

Jeffamine M-2070

$MeO(CH_2CH_2O)_{45}CH_2CHNHCOCl + NeuNAc\alpha 1\text{-}3Gal\beta 1\text{-}4GlcNAc \rightarrow$

Sialyl Lewis X 3

Fucα1

$MeO(CH_2CH_2O)_{45}CH_2CHNHCO$

$CH_3$ 6

$NeuNAc\alpha 1\text{-}3Gal\beta 1\text{-}4GlcNAc$

3

Fucα1

Jeffamine M-2070, (388mg, 0.187mmol), is dissolved in 1mL of acetonitrile. To which triphosgene (18.6mg, 0.187mmol) is added slowly, the reaction is stirred for 20 minutes. 1μL of this solution is added to 3' Sialyl Lewis X. In a separate vial pyridine, (14.8mg, 0.187mmol) is dissolved in 100μL of acetonitrile. 0.1μL of this solution is added to 3' Sialyl Lewis X (0.14mg, 0.17μmol). Reaction is allowed to stand for 24h at room temperature.

Example 7

$C_nF_{2n+1}CH_2CH_2(OCH_2CH_2)_9OH + CCl_3OCOCCl_3 \rightarrow C_nF_{2n+1}CH_2CH_2(OCH_2CH_2)_9OCOCl$

PEG Telomer B

$C_nF_{2n+1}CH_2CH_2(OCH_2CH_2)_9OCOCl + NeuNAc\alpha 1\text{-}3Gal\beta 1\text{-}4GlcNAc \rightarrow$

Sialyl Lewis X 3

Fucα1

$C_nF_{2n+1}CH_2CH_2(OCH_2CH_2)_9OCO$

6

$NeuNAc\alpha 1\text{-}3Gal\beta 1\text{-}4GlcNAc$

3

Fucα1

PEG Telomer B, (140mg, 0.187mmol), is dissolved in 1mL of acetonitrile. To which triphosgene (18.6mg, 0.187mmol) is added slowly the reaction is stirred for 20h. To this solution pyridine, (14.8mg, 0.187mmol) is added. 1μL of this solution is added to 3' Sialyl Lewis X, (0.14mg, 0.17μmol). The reaction is allowed to stand for 24h at room temperature.

Example 8

$C_8F_{17}SO_2N(CH_2CH_3)_2(CH_2CH_2O)_{14}H+CCl_3OCOCCl_3\rightarrow$

Fluorad F-170C

$C_8F_{17}SO_2N(CH_2CH_3)_2(CH_2CH_2O)_{14}COCl$

$C_8F_{17}SO_2N(CH_2CH_3)_2(CH_2CH_2O)_{14}COCl$+NeuNAcα1-3Galβ1-4GlcNAc→

Sialy Lewis X 3

Fucα1

$C_8F_{17}SO_2N(CH_2CH_3)_2(CH_2CH_2O)_{14}CO$

6

NeuNAcα1-3Galβ1-4GlcNAc

3

Fucα1

PEG Telomer B, (140mg, 0.187mmol), is dissolved in 1mL of acetonitrile. To which triphosgene (18.6mg, 0.187mmol) is added slowly the reaction is stirred for 20h. To this solution, pyridine, (15.8mg, 0.187mmol) is added. 1μL of this solution is added to 3' Sialyl Lewis X, (0.14mg, 0.17μmol). The reaction is allowed to stand for 24h at room temperature.

Example 9

This Example shows the synthesis of cyclic peptides with disulfide bridges.

Peptide syntheses are performed by the solid-phase method (Steward, J. M. and Young, J. D., Solid Phases Peptide Synthesis, 2nd ed.; Pierce Chemical Co., Rockford, IL., 1984), utilizing an automated synthesizer (Applied Biosystems, Inc. Model 431A). Carboxamide peptides are synthesized with p-methylbenzhydrylamine (pMBHA) resin and peptides with C-terminal acids were synthesized with chloromethylated resin. N-terminal tert-butyloxycarbonyl protection is employed for all amino acids. Boc-Arg(Tos)OH, Boc-Asp (OcHx)-OH, Boc-Cys(4-MeBzl)-OH, Boc-Gly-CH, BocHpa-OH, Boc-Lys (CZ)-OH, Boc-Pen (4-MeBzl)-OH, Boc-DPen(4MeBzl)-OH, Boc-Pro-OH, and Boc-Tyr(Me)-OH may be obtained from Bachem Inc. (Torrance, CA). Pmp(4-MeBzl)-OH and Boc-Tyr(n-Butyl)-OH may be synthesized according to the procedures of Yim. C.F.N. and Huffman, W.F. *Int.J. Peptide Protein Res.* (1983) 21:568, and Solar, S. L. and Schumaker, *R J. Org. Chem.,* (1966) 31:1996, respectively. Dicyclohexylcarbodiimide and hydroxybenzyltriazole may used in the coupling reactions, which can monitored by the ninhydrin test.

For the preparation of peptides with N-terminal acetyl, the peptides are acetylated using a mixture of acetic anhydride (20 eq.) and diisopropylethylamine (20 eq.) in N-methyl pyrrolidone.

The peptides are removed from resin and deprotected with anhydrous hydrogen fluoride (HF;10 mL/g of resin-bound peptide) containing anisole (1 mL/g) at 0°C for 60 minutes. After the evaporation of HF, the residue is washed with anhydrous ether, and the crude peptides are extracted with water or 15W aqueous acetic acid. The aqueous fractions are combined and lyophilized.

The crude acyclic peptide is dissolved in 0.1M ammonium bicarbonate (0.5 mg/mL) and stirred open to the air. The course of the reaction may be monitored via HPLC. After cyclization was complete (several hours to several days), the solution is filtered and purified via preparative RP-HPLC on a $C_{18}$ silica gel column such as Waters DeltaPak, 15ym, 300A, 47x300 mm. Elution may be done with a linear acetonitrile gradient (0-40%) with a constant concentration of trifluoroacetic acid (0.1k, v/v) over 20 minutes at a flow rate of 40 mL/min.

The purified peptides may be analyzed by analytical reversed-phase HPLC on C-18 Columns (Vydac, 5 ym, 300A, 4.5 x 250 mm). The solvent system used for analytical HPLC may be a binary system, water containing 0.1% TFA and acetonitrile containing 0.1% TFA as the organic modifier. Suitable solvent programs may involve linear gradients as follows: (1) 0% to 40% acetonitrile over 15 min. with flow rate of 1.5 mL/min.; (2) 0% to 50% acetonitrile over 15 min.

with flow rate of 1.5 mL/min.; (3) 0% to 60% acetonitrile over 15 min. with flow rate of 1.5 mL/min.

Example 10

This example shows the synthesis of cyclic peptides with lactam bridges.

The protected peptide resins are synthesized with p-methylbenzhydrylamine (pMBHA) resin. The formation of the lactam on the resin can be by the methodology of Felix and co-workers. Felix, et al. *Int. J. Peptide Protein Res.* (1988) 31:231; Felix, A.F. et al. Int. *Int.J. Peptide Protein Res.* (1988) 32:441. This methodology uses $N^{\alpha}$-Boc-amino acids together with OFm side-chain protection for Asp and Glu. Asp and Glu are introduced with Boc-Asp(OFm)-OH and Boc-Glu(OFm)-OH. After coupling the last amino acid, the OFm protecting groups are selectively removed by treating the peptide resin in 50W piperidine in DMF (Dimethylformamide) for 1 h. The peptide resin is washed with DMF (3 x 40 mL), DCM (dicholoromethane) (3 x 40 mL), suspended in 15 mL of DMF, and mixed with a 6-fold excess of BOP reagent in the presence of an 8-fold excess of diisopropylamine (DIEA) for 5 h.

After the cyclization, the peptides are suitably removed from resin and deprotected with anhydrous Hydrogen fluoride (HF; 10 mL/g of resin-bound peptide) containing anisole (1 mL/g) at 0°C for 60 minutes. After the evaporation of HF, the residue is washed with anhydrous ether, and the crude peptides are extracted with water or 15% aqueous acetic acid. The aqueous fractions are combined and lyophilized.

The crude peptides are purified via preparative RP-HPLC on a $C_{18}$ silica gel column (Waters Delta-Pak, 15 μm, 300A, 47x300 mm) eluting with a linear acetonitrile gradient (0-40%) with a constant concentration of trifluoroacetic acid (0.1%, v/v) over 20 minutes at a flow rate of 40 mL/min.

The purified peptides are then analyzed by analytical reversed-phase HPLC on C-18 Columns (Vydac, 5 μm, 300A, 4.5x250 mm). The solvent system used for analytical HPLC can be a binary system, water containing 0.1% TFA and acetonitrile containing 0.1% TFA as the organic modifier, and the solvent programs may involve linear gradients as follows: (1) 0% to 40% acetonitrile over 15 min. with flow rate of 1.5 mL/min.; (2) 0% to 50% acetonitrile over 15 min. with flow rate of 1.5 mL/min.; (3) 0% to 60% acetonitrile over 15 min. with flow rate of 1.5 mL/min.

Example 11

These examples (11-14) show the preparation of conjugants of RGD peptides with various surfactants as identified.

Arg-Gly-Asp (RGD), (10mg, 28.8 μmol), is dissolved in 400 μL DMSO with 15 μL pyridine in a 6x50mm culture tube equipped with a flea stirbar and topped with a septa. In a separate culture tube, Peg Telomer B, (33mg, 36.7 μmol) is dissolved in 60 μL dichloroethane, In a separate culture tube, triphosgene, (38mg, 128 μmol) is dissolved in 110 μL. 10 μL of the triphosgene solution is transferred to the Peg Telomer B solution. A deep yellow color ensues and allowed to stir for 30 min. All of the PEG Telomer B solution is added to the RGD solution, color momentarily disappears. The reaction mixture is heated to 50°C for 16h. TLC shows disappearance of Peg Telomer B starting material. The surfactant/ligand combination is precipitated by addition of methylene chloride.

Example 12

Arg-Gly-Asp-Ser (RGDS), (10mg, 23 μmol), is dissolved in 400 μL DMSO with 12 μL pyridine in a 6x50mm culture tube equipped with a flea stirbar and topped with a septa. In a separate culture tube, Peg Telomer B, (27mg, 29 μmol) is dissolved in 60 μL dichloroethane. In a separate culture tube, triphosgene, (30mg, 102 μmol) is dissolved in 88 μL. 10 μL of the triphosgene solution is transferred to the Peg Telomer B solution. A deep yellow color ensues and allowed to stir for 30 min. All of the Pet Telomer B solution is added to the RGDS solution, color momentarily disappears. The reaction is heated to 50°C for 16h. TLC shows disappearance of Peg Telomer B starting material. The surfactant/ligand combination is precipitated by addition of methylene chloride.

Example 13

Gly-Arg-Gly-Asp (GRGD), (10mg, 22 μmol), is dissolved in 400 μL DMSO with 12 μL pyridine in a 6x50mm culture tube equipped with a flea stirbar and topped with a septa. In a separate culture tube, Peg Telomer B, (26 mg, 29 μmol) is dissolved in 60 μL dichloroethane. In a separate culture tube, triphosgene, (30 mg, 102 μmol) is dissolved in 88 μL. 10 μL of the triphosgene solution is transferred to the Peg Telomer B solution. A deep yellow color ensues and allowed to stir for 30 min. All of the Peg Telomer B solution is added to the GRGD solution, color momentarily disappears. The reaction is heated to 50°C for 16h. TLC shows disappearance of Peg Telomer B starting material. The surfactant/ligand combination is precipitated by addition of methylene chloride.

Example 14

Echistatin, (0.1 mg, 18.5 nmol), is dissolved in 4 μL DMSO with 0.15 μL pyridine in a 6x50 mm culture tube equipped with flea stirbar and topped with a septa. In a separate culture tube, Peg Telomer B, (33 mg, 36.7 μmol) is dissolved in 1 mL dichloroethane. In a separate culture tube, triphosgene, (38 mg, 128 μmol) is dissolved in 110 μL. 10 μL of the triphosgene solution is transferred to the Peg Telomer B solution. A deep yellow color ensues and allowed to stir for 30 min. 1μL of the Peg Telomer B solution is added to the Echistatin solution. The reaction is heated to 50°C for 16h. The surfactant/ligand combination is precipitated by addition of methylene chloride.

Once formed, the CAM ligand/surfactant conjugate is incorporated into a formulation which permits the ligand to act at the surface between the dispersed and continuous phases of the colloidal dispersion which is the ultrasound contrast agent of the preferred embodiment. Alternatively, the ligand would be conjugated at the surface of a proteinaceous microsphere to attract the gas containing microsphere to the surfaces of cells as desired by a particular application.

The quantity of ligand is determined simply by substitution into known formulations. That is, the conjugated CAM ligand/surfactant is used in quantities as necessary to effect cell adhesion of the surfactant.

In a specific use of the invention one possible formulation would be a colloidal dispersion formed from the combination of a medical grade, highly purified PEG Telomer B surfactant at 0.3% w/v, 30% sucrose w/v and 2% dodecafluoropentane w/v at pH of 7. In conjunction with or as a supplement, the surfactant/CAM ligand conjugates of Examples 11-14 are included with the PEG Telomer B surfactant. Generally in order to ensure that the conjugate is found only at the interface of the dispersed and continuous aqueous phase, amounts of the total surfactant and surfactant/CAM ligand conjugate would be the minimum necessary to form a stable dispersion. As is known, these components would be subjected to low shear mixing, then comminuted in a Microfluidizer at 4°C to form a milky emulsion.

Specific CAM ligands which can be incorporated into the present invention are known to target a defined group of cells on which corresponding CAMs are expressed. Many specific types of such interactions have been identified in the technical and commercial literature (see, for example, the summary produced by R & D Systems, 614 McKinley Place N.E. Minneapolis MN 55143 800-343-7475, incorporated herein by reference) and are readily available to the skilled artisan.

Having identified the cell type and CAM expressed thereon, the contrast agent designer may then match a specific surfactant with a compatible CAM ligand to formulate an agent which will be targeted to that cell type. Sites of ischemia, such as following trauma, mycardial infarction or stroke produce intercellular adhesion molecules (ICAMs). Ultrasound contrast media incorporating integrins as either part of a surfactant used therein, or associated with the surface of a proteinaceous microsphere, would selectively bind to the ICAM and enhance the ultrasound contrast signal available from such area.

Example 15

This example describes a process for studying in vitro sequestering of dodecafluropentane (DDFP) particles.

Emulsion particles are fragile fluid structures, constantly in equilibrium with the surrounding media and themselves. Attaching an adhesive molecule to the surface of a particle does not assure adhesion of a particle to another surface. Furthermore, most emulsions are created to repel other surfaces through steric or electrostatic forces. Since there are no covalent bonds linking a surfactant adhesion molecule to its particle, contact between an adhesive pair, might very well lead to extraction of the surfactant adhesion molecule from its particle with no adhesion of the particle to the surface. In order to test the feasibility of site directed ultrasound contrast, we developed an *in vitro* isolation procedure using the avidin-biotin interaction as a means of accomplishing this.

The avidin-biotin interaction was chosen because of its speed, $K_d$=1.3 x$10^{-15}$ (the strongest non-covalent biological interaction known), stability in an aqueous environment, and insensitivity to most other compounds make it ideal for study with emulsions, which undergo shift from liquid to gas phase just above room temperature. We coupled biotin to a perfluorinated fragment and avidin to a large latex particle (See FIG. 1). This allows the complex to be removed by filtration and quantified. The process can be summarized as follows:

$$C_7F_{15}CH_2NH_2 + \text{NHS-Biotin} \rightarrow \text{Biotin-NHCH}_2C_7F_{15},$$

Biotin-$NHCH_2C_7F_{14}$ + surfactant + DDFP + homogenization to form a surfactant/DDFP particle and biotin conjugate. The surfactant/DDFP particle and biotin conjugate is then mixed with avidin coupled microspheres. See FIG. 1.

Latex microspheres, 1μ, carboxylate modified, Avidin-labeled, 1% solids, red fluorescent (580/605) (Molecular Probes, Inc., Eugene Oregon), 0.1mL, are loaded on to a 0.45μ 25 mm Acrodisc® filter, (Gelman Sciences), with 5 mL deionized water. Microspheres are backflushed into 0.1mL 1.94% DDFP (2.35MG) emulsified with 0.05% $C_7F_{15}CH_2NH$-Biotin and PEG Telomer B purified as described in Example 1 in 30% sucrose in a 1mL syringe. The mixture of emulsion and latex particles is agitated for 60 seconds by repeated inversion. The mixture is slowly pushed

back into the filter and 5 mL deionized water is slowly passed through the filter to wash through the uncomplexed DDFP particles. The collected filtrate is removed and the filter is immediately backflushed with 0.75mL deionized water into the 5mL syringe and put into a sealed 20mL headspace vial containing 0.5mL isopropanol and weighed, 0.7480g. The sample was analyzed quantitatively for DDFP by GC and found to contain 0.84mg. (36% recovery).

A control experiment was run using the above procedure repeated with 0.1mL 1.94% DDFP emulsified only with a non-ionic surfactant in 30% sucrose. A 0.5337g backflush was recovered which was analyzed to contain only 0.09mg, (4.3% recovery). This in vitro study indicates that DDFP microparticles could be sequestered by a biological-based interaction just as would occur in the body

Example 16

This example shows the selection of a surfactant for the formation of a stable non-toxic emulsion containing the surfactant/ CAM ligand conjugate for parenteral administration.

We have found that for an stable emulsion of a 2% volatile oil using 0.2-0.4% surfactants, in 10-40% sucrose a purified composition of non-ionic surfactant having the formula $C_nF_{2n+1}(CH_2CH_2O)_xH$ where n=6-12, average = 9 and x=2-16, average = 10 is preferred.

Examples of non-ionic fluorocarbon surfactants which generally give less stable emulsions under the above conditions include Afilan OTN, and Zonyl FSN-100.

Thus, although the invention has been described in some respects with reference to specified preferred embodiments thereof, many variations and modifications will be readily apparent to those skilled in the art. It is, therefore, the intention that the following claims not be given a restrictive interpretation but should be viewed to encompass such variations and modifications that may be routinely derived from the subject matter herein disclosed.

## Claims

**1.** A biocompatible ultrasound contrast agent for use in an animal having a body temperature T comprising:

a chemical having a sufficiently high vapor pressure that a portion of said chemical is a gas at the temperature T,
a liquid carrier, and
a surfactant or albumin carrier wherein said surfactant or albumin includes a CAM ligand.

**2.** The ultrasound contrast agent of claim 1 wherein said CAM ligand binds to the GPIIb/IIIa receptor.

**3.** The ultrasound contrast agent of claim 1 wherein said CAM ligand is an KGD or RGD containing molecule.

**4.** The ultrasound contrast agent of claim 3 wherein said KGD or RGD containing molecule is selected from the group consisting of: trigramin, kistrin, albolabrin, elegantin, jarahagin, jaracetin, contortrostatin, multisquamatin, echistatin, eristocophin, eristostatin, albolabrin, applagin, barbourin, basilicin, batroxostatin, cerastin, ceraberin, cotiarin, crotatroxin, durissin, elegantin, flavoridin, halysin, jararacin, kistrin, lachesin, lutomin, moloosin, torgeminin, trigramin, viridin, bitistain, decorisn, mambin, ornatio E and halystatin.

**5.** The ultrasound contrast agent of claim 3 wherein said RGD containing peptide is a cyclic molecule having the structure:

$$X_1\ X_2\ X_3\ X_4\ G\ D\ X_5\ X_6\ X_7\ X_8$$

wherein $X_1$ and $X_8$ are 0 to 20 amino acids;
$X_2$ is 0 or an amino acid capable of forming a lactam bridge;
$X_3$ is 0 to 10 amino acids;
$X_4$ is a positive amino acid;
$X_5$ is a hydrophobic amino acid except leucine;
$X_6$ is a positively charged amino acid; and
$X_7$ is an amino acid capable of forming a lactam bridge.

**6.** A biocompatible ultrasound contrast agent for use in an animal having a body temperature T comprising:

a chemical having a sufficiently high vapor pressure that a portion of said chemical is a gas at the temperature T,

a liquid carrier,
and at least one surfactant carrier wherein said surfactant includes a CAM ligand.

**7.** The ultrasound contrast agent of claim 6 further comprising a second surfactant.

**8.** The ultrasound contrast agent of claim 7 wherein the second surfactant comprises a non-ionic surfactant having the formula $C_nF_{2n+1}(CH_2CH_2O)_xH$ wherein n=6-12 and x=2-16.

**9.** The ultrasound contrast agent of claim 8 wherein said CAM ligand is an RGD or KGD containing molecule.

**10.** A biocompatible ultrasound contrast agent for use in an animal having a body temperature T comprising:

a chemical having a sufficiently high vapor pressure that a portion of said chemical is a gas at the temperature T,
a liquid carrier,
a first surfactant including an RGD containing peptide, and
a second surfactant wherein the second surfactant comprises a non-ionic surfactant having the formula $C_nF_{2n+1}(CH_2CH_2O)_xH$ wherein n=6-12 and x=2-16.

**11.** The ultrasound contrast agent of claim 10 wherein said first surfactant is PEG Telomer B.

**12.** The ultrasound contrast agent of claim 11 wherein said RGD peptide is selected from the group consisting of RGD, RGDS, GRGD and echistatin.

**13.** The ultrasound contrast agent of claim 12 wherein said second surfactant is PEG Telomer B.

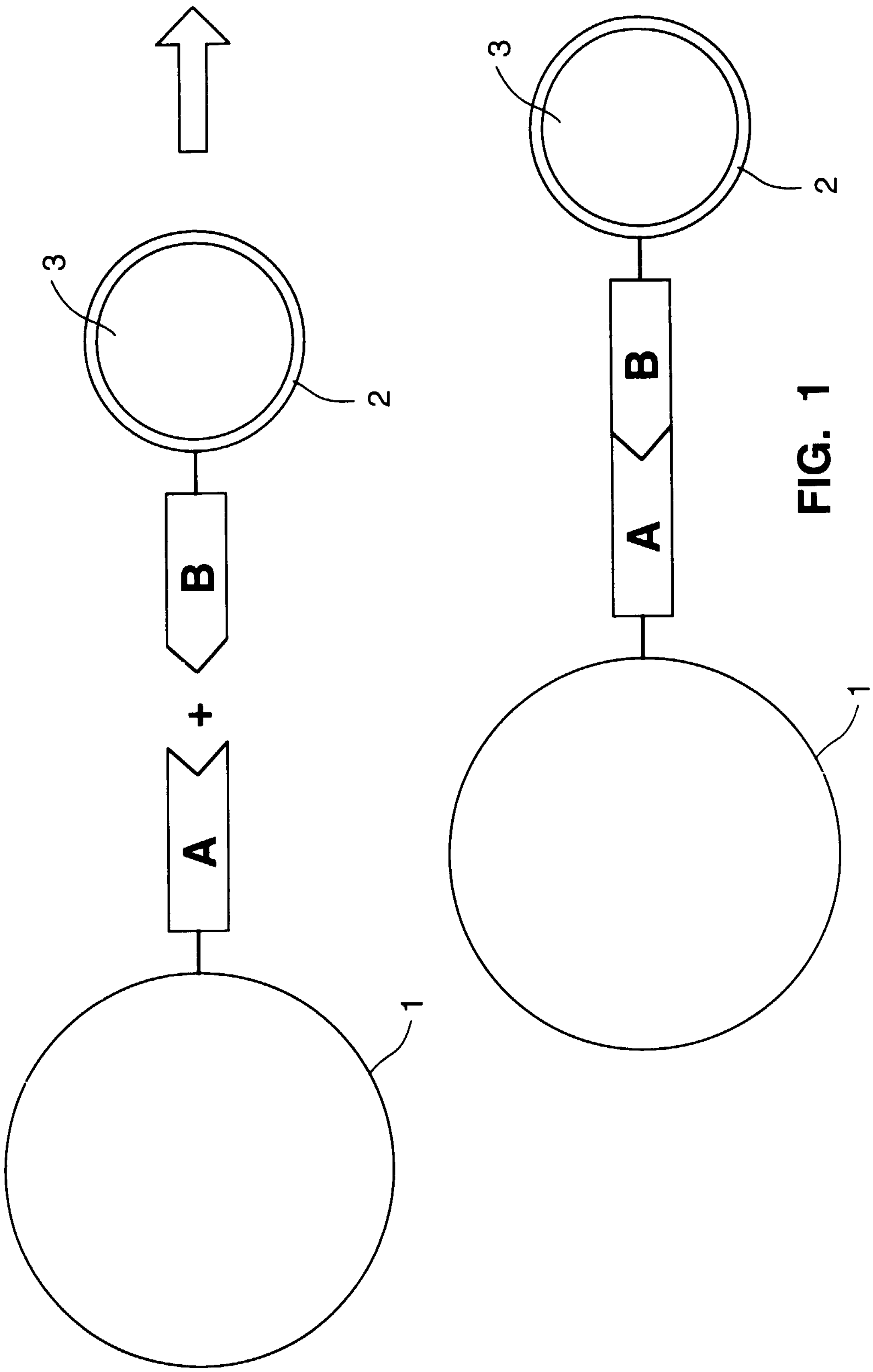


FIG. 1